# EUROPEAN PATENT APPLICATION

(11) **EP 3 211 081 A1**
(43) Date of publication of application: **30.08.2017**
(21) Application number: 16000468.5
(22) Date of filing: 26.02.2016
(51) Int. Cl.: C12N 15/113, A61K 31/7125, A61P 29/00, A61P 35/00

(54) **NOVEL APPROACH FOR TREATING INFLAMMATORY DISORDERS**

(71) Applicant: Secarna Pharmaceuticals GmbH & Co. KG, 82152 Planegg/Martinsried (DE)
(72) Inventor: Jaschinski, Frank, 93083 Obertraubling (DE); Ksenija, Schirduan, 80469 München (DE)
(74) Representative: Virnekäs, Bernhard

(57) **Abstract**

The present invention relates to a novel approach for treating inflammatory disorders and other diseases, which is based on targeting ROR gamma t mRNA. The invention is directed to oligonucleotides comprising 10 to 20 modified or unmodified nucleotides complementary to specifically selected regions of the RORC, transcript variant 2 mRNA (RORC2), which codes for the ROR gamma t protein.

## Description

### FIELD OF THE INVENTION

The present invention relates to a novel approach for treating inflammatory disorders and other diseases, which is based on targeting ROR gamma t mRNA. The invention is directed to oligonucleotides comprising 10 to 20 modified or unmodified nucleotides complementary to specifically selected regions of the RORC, transcript variant 2 mRNA (RORC2), which codes for the ROR gamma t protein.

### BACKGROUND OF THE INVENTION

Th17 cells are a subset of T helper cells which have been associated with several chronic inflammatory and autoimmune diseases. Upon polarization and activation Th17 cells produce highly proinflammatory molecules like cytokines IL-17A, IL-17F, IL-21, IL-22 and chemokines CXCL8 and CCL20. Furthermore, Th17 cells produce effector molecules like GM-CSF as well as provide help to B cells, induce germinal center formation and class switching.

The effector mechanisms and exacerbating Th17 responses are associated with the pathogenesis of acute and chronic inflammatory diseases like psoriasis, rheumatoid arthritis (RA), multiple sclerosis (MS), systemic lupus erythematosus (SLE), diverse phenotypes of inflammatory bowel diseases (IBD), asthma and allergy.

In rheumatoid arthritis the role of Th17 has been confirmed in several mouse models as well as in patient's material. High frequency of IL17 producing cells has been found in diseased synovium, and intra-articular administration of IL17 in collagen-induced arthritis models has led to worsening of RA symptoms. Furthermore, IL17RA deficient mice develop only very mild forms of RA. To date, anti-IL17A and anti-IL17RA monoclonal antibodies have been shown protective from progressive RA in humans and are approved as therapeutics.

In psoriasis, psoriatic arthritis as well as plaque psoriasis, chronic activation of Th17 cells and pro-inflammatory response against skin autoantigens as well as skin microbiota is the major cause of pathology. Respective patients' skin biopsies show high levels of IL17, IL23, IL6 and IL12, which represent the proinflammatory mixed Th1/Th17 phenotype. Additionally, Th17 cells in psoriatic plaques produce CCL20 and recruit more Th17, mast cells & neutrophils to prolong and elevate the response. Further, Th17 plays a role in inflammation processes that are part of several other skin phenotypes like acne, dermatomyositis and scleroderma.

Beside the skin diseases, Th17 cells play also an important role in further mucocutaneous inflammatory diseases such as chronic, allergic and steroid-resistant asthma, chronic obstructive pulmonary disease (COPD) and inflammatory bowel diseases, like Crohn's disease, enteritis and ulcerative colitis. IL17A has been found in high levels in sputum of severe asthma patients as well as of COPD patients. IL17 is potently induced via aryl-hydrocarbon receptor (AhR), a target of cigarette smoke ingredients and several pollutants.

The pathology of IBD is an intensively studied field and convincing data, despite diversity of models used, show the importance of Th17 cells. In Crohn's disease patients experience reoccurring disease phases with increased Th17 frequencies. The strong Th17-driven autoimmune response might be linked to the IL23R polymorphism, and the overshooting host defense against gut microbiota is based on a multiplicity of Th17-mediated mechanisms. Nevertheless, the inhibition of IL17 was not shown beneficial in Crohn's disease, which mirrors the diversity of IBD phenotypes in the patient population.

One of the first diseases that was shown to be Th17-driven was multiple sclerosis and although the complexity of the disease increases, Th17 cells still remain in the central role. Diverse cell types from the brain biopsies of MS patients showed IL17A overexpression and extensive infiltration of lymphocytes. Furthermore, the peripheral phenotype of MS seems also to be Th17-dependent, as these cells have been shown to provide B cell help and may induce autoantibody production.

As in MS, similar involvement of Th17 cells has been shown in another autoimmune phenotype - systemic lupus erythematosus ("SLE"). This complex and diverse disease worsens with increase of Th17 cell numbers. Major effect of Th17 in SLE was observed in progressive vasculitis and endothelial dysfunction. This effect is not only characteristic to SLE, but also to Behcet's disease, uveitis and Sjögren syndrome. IL 17-producing cells have been found to accumulate in vessel walls of autoimmune vasculitis patients and recruit other proinflammatory cells, like neutrophils and macrophages. IL17 has prothrombic and procoaggulant effects on human endothelial cells.

Concomitantly with accumulation of Th17 in vasculitis there is a positive correlation between Th17 frequency and frequency of aortic lesions in coronary atherosclerosis. Here, increase in Th17 levels lead to increase in size of aortic lesions and frequency of cardiac infarction.

Beside rheumatoid arthritis, Th17 plays a role in another chronic inflammatory joint and spine disease, Morbus Bechterew. Here, during ongoing tendon inflammation Th17 and mast cells both produce IL17 in the synovium and drive ectopic bone formation and joint stiffness. The ongoing inflammation is fairly treatable with anti-TNF alpha therapy but the bone reformation and ankyloses might be Th17 dependent.

One yet partially explored field of Th17 cells is neuropathic pain. There is accumulating data that diverse neuropathic and chronic unspecific low back pain correlate with the disrupted balance between Th17 and regulatory T cells.

Taken together, inhibition of Th17 development and function appears to clearly be a promising target for the treatment of the above-mentioned diseases.

The master transcription factor of Th17 cells is ROR gamma t. Retinoic acid receptor-related orphan receptors (RORs) are members of the steroid hormone nuclear receptor family. The ROR transcription factor subfamily consists of three members: ROR alpha (RORA), ROR beta (RORB) and ROR gamma (RORC). Each member is expressed as independent gene and binds as monomer to genomic response elements. Through alternative splicing and differential promoter usage each ROR member generates variants that have different tissue expression patterns and that regulate different targets. RORC has two isoforms that arise from differential promoter usage and that differ in the first exon. These two transcript variants are annotated as: NM_005060 for ROR gamma (RORC1) and NM_001001523 for ROR gamma t (RORC2). While ROR gamma is expressed in a variety of tissues either constitutively or under circadian rhythm, ROR gamma t expression is limited to the cells of the immune system. The highest expression of ROR gamma was shown for skeletal muscle, kidney and liver while ROR gamma t expression peaks in the developing double positive thymocytes, T helper lineage 17 cells (Th17), lymphoid-tissue inducer cells (LTi) and several intraepithelial lymphoid cell (ILCs) subpopulations. ROR gamma t has a critical role in thymopoiesis as it reduces Fas expression and IL2 dependency, rescuing the thymocytes from the activation-induced cell death during thymic selection. Further, ROR gamma t is important for the function of LTis and development of secondary lymphoid tissues.

Thus, inhibition of Th17 development and function through the inhibition of the master differentiation factor, ROR gamma t, appears to be a viable option.

One possible approach for inhibiting the Th17-driven pathologies is to inhibit the effector functions of already present Th17 as well as interfere with the new differentiation of naïve T cells towards Th17 lineage by blocking ROR gamma t. However, ROR gamma t is an intracellular molecule and cannot be reached by therapeutic antibodies. Furthermore, the currently available ROR gamma and gamma t small molecule antagonists target the ligand or DNA binding domains that are identical between the both molecules. As previously stated, ROR gamma is ubiquitously expressed and has roles in many central physiological processes. The most studied role of ROR gamma is the circadian regulation of lipid and sugar metabolism in liver and skeletal muscle. The absence of ROR gamma leads to imbalance of liver metabolic function, metabolic syndrome, insulin resistance and obesity. Additionally, several studies have evidenced an important role of ROR gamma in cancer. ROR gamma deficiency in mice leads to a high incidence of thymic lymphomas with frequent liver and spleen metastasis. Thus, there is a high an unmet need for ROR gamma t-specific inhibitors.

One alternative approach for inhibiting the Th17-driven pathologies is to knock down the expression of ROR gamma t on the nucleic acid level. Such targeting has several important advantages, since in comparison with the available small molecule therapeutics, targeting on the nucleic acid level may be adjusted to target the ROR gamma t transcript exclusively, thus having no effects on ROR gamma. Small molecules currently in development do not have this level of specificity which may lead to adverse side-effects with these drug candidates. However, the initial approach of using DNAzymes based on the 10-23 motif, which had already been successfully applied to the inhibition of GATA-3 (see WO 2005/033314) did not result in an inhibition of the expression of RORC2 relative to untreated control in HDLM2 cells by at least 50% (see Example 2).

Thus, therapeutic agents which are able to specifically inhibit the expression of ROR gamma t intracellularly are essential for the treatment of Th17-driven diseases, in particular inflammatory disorders of various organs. Hence, there is still a high scientific and medical need for therapeutic agents, which reduce or inhibit RORC2 expression and/or activity. Particularly, there is a longstanding need for oligonucleotides, which specifically interact and thus, reduce or inhibit the expression of RORC2, as well as oligonucleotides, which specifically inhibit RORC2, without causing any (severe) side effects.

### SUMMARY OF THE INVENTION

Despite the negative results obtained with a DNAzyme based approach, the present inventors surprisingly identified that a rather similar approach of using certain antisense constructs was able to achieve the inhibition of the expression of RORC2 relative to untreated control in HDLM2 cells by at least 50%, with several candidates achieving inhibition of more than 80%. This finding was unexpected and neither taught nor suggested by the prior art.

Thus, in a first aspect, the present invention relates to an oligonucleotide consisting of from 10 to 20 nucleotides, particularly from 15 to 18 nucleotides, wherein the sequence of said oligonucleotide corresponds to the antisense strand of the RORC2 nucleic acid coding sequence of SEQ ID NO. 1, wherein one or more nucleotide(s) of the oligonucleotide is/are optionally modified, and wherein said oligonucleotide inhibits the expression of RORC2 relative to untreated control in HDLM2 cells by at least 50%.

In a second aspect, the present invention relates to a pharmaceutical composition comprising the oligonucleotide according to the present invention.

In a third aspect, the present invention relates to the oligonucleotides or the pharmaceutical composition according to the present invention for use in a method of preventing and/or treating a disease or disorder selected from the list of: an acute inflammatory disease, a chronic inflammatory disease, a neurodegenerative disease, a malignant tumor, and a benign tumor.

### BRIEF DESCRIPTION OF THE DRAWINGS

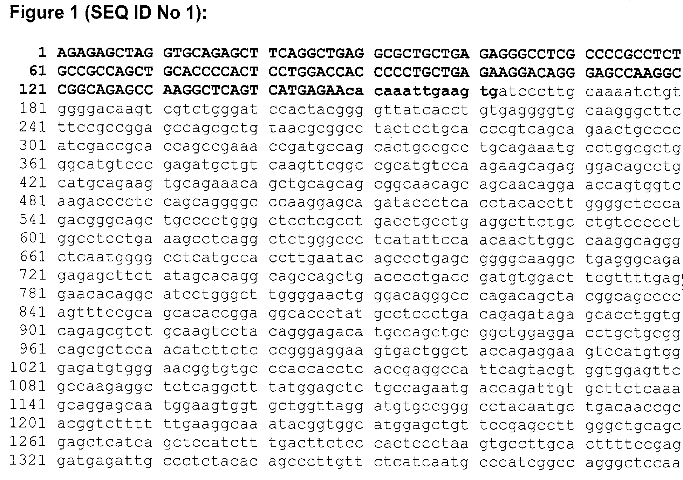

**Figure 1** shows the complete RORC, transcript variant 2 mRNA (NM_001001523.1). In capital letters, the region unique to RORC2 (ROR gamma t) and not shared with RORC1 is given. Given that at least 3 mismatches to RORC1 are required, the usable space for 17mer oligonucleotides specific for only RORC2 is indicated in bold letters. Bioinformatic screening was performed within this area in order to preserve specificity of oligonucleotides to only RORC2.

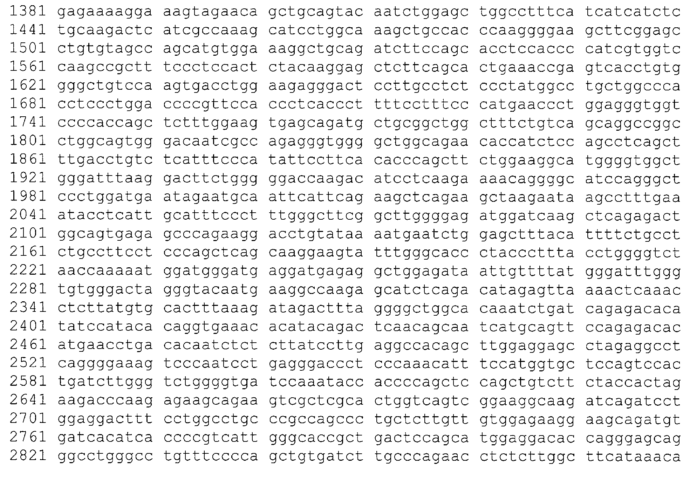

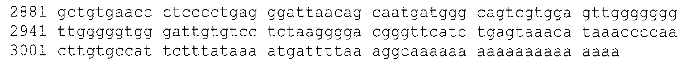

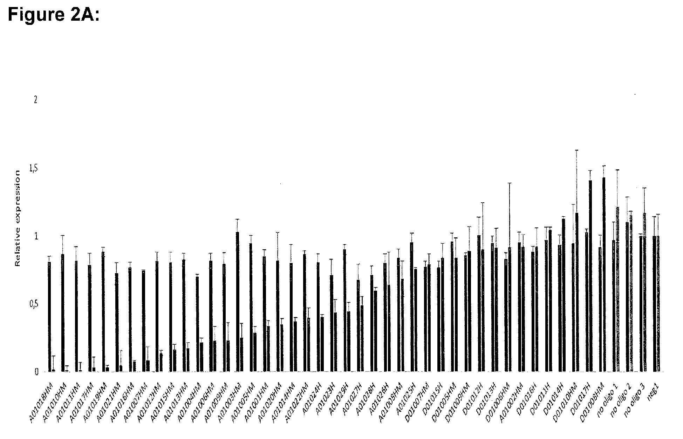

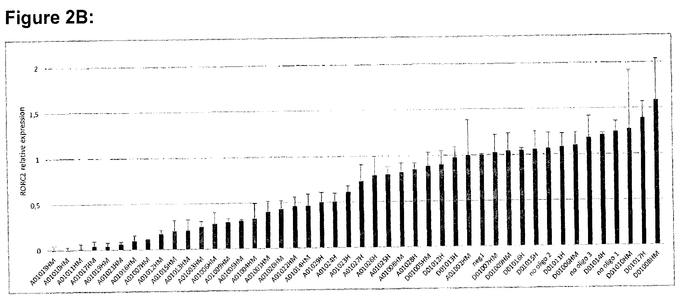

**Figure 2A** shows the result of a first oligonucleotide screen for ROR gamma t inhibition in HDLM2 cells, as described in Example 3: first 42 pairs of bars from the left: HPRT1 (left, dark bars), RORC2 (right, brighter bars) for antisense oligonucleotides (identifiers "A....") or DNAzymes (identifiers "D....")); pairs of bars no. 2, 3, and 4 from the right: HPRT1 (left, dark bars), RORC2 (right, brighter bars for no oligonucleotide controls); pairs of bars on the left: HPRT1 (left, dark bar), RORC2 (right, brighter bar for negative control oligonucleotide). **Figure 2B** shows the inhibition of expression of RORC2 relative to the expression of untreated control in HDLM2 cells, normalized to HPRT1.

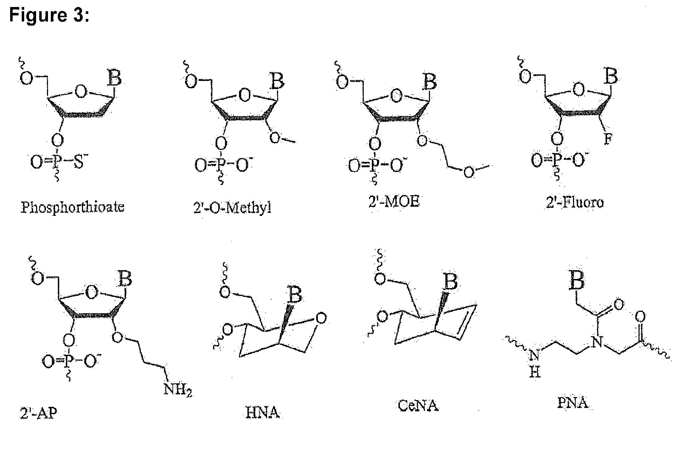

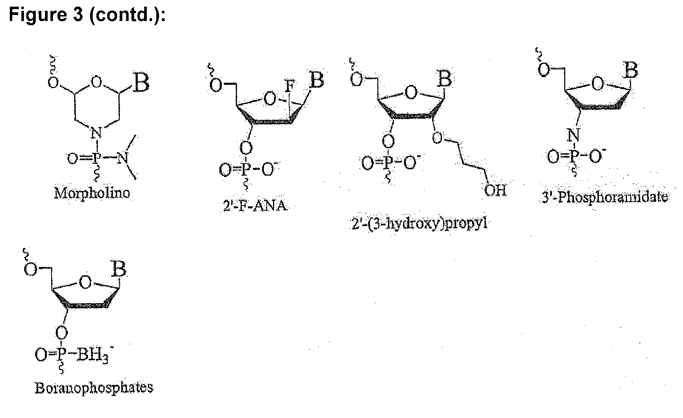

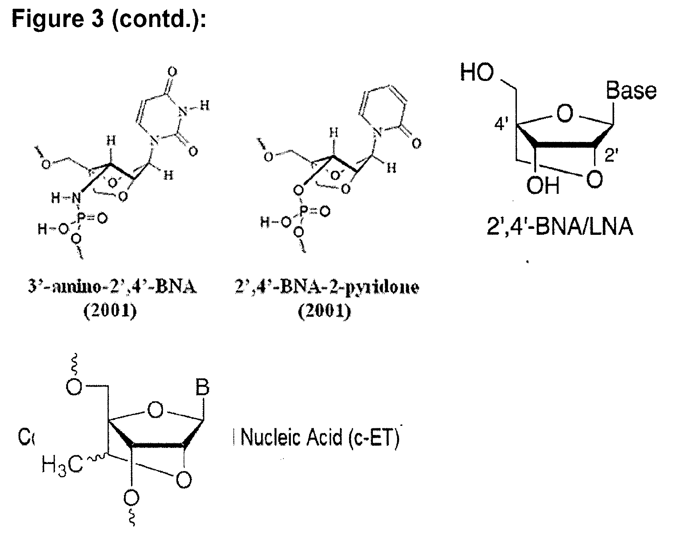

**Figure 3** shows a number of different modified nucleotides that may be used in the context of the present invention.

### DETAILED DESCRIPTION OF THE INVENTION

Thus, in a first aspect, the present invention relates to an oligonucleotide consisting of from 10 to 20 nucleotides, particularly from 15 to 18 nucleotides, wherein the sequence of said oligonucleotide corresponds to the antisense strand of the RORC2 nucleic acid coding sequence of SEQ ID NO. 1, wherein one or more nucleotide(s) of the oligonucleotide is/are optionally modified, and wherein said oligonucleotide inhibits the expression of RORC2 relative to untreated control in HDLM2 cells by at least 50%.

In particular embodiments of the present in invention, said oligonucleotide inhibits the expression of RORC2 relative to untreated control in HDLM2 cells by at least 75%, particularly by at least 80%, more particularly by at least 85%.

In particular embodiments of the present in invention, one or more nucleotide(s) in said oligonucleotide are modified.

A nucleotide forms the building block of an oligonucleotide, and is for example composed of a nucleobase (nitrogenous base, e.g., purine or pyrimidine), a five-carbon sugar (e.g., ribose, 2-deoxyribose, arabinose, xylose, lyxose, allose, altrose, glucose, mannose, gulose, idose, galactose, talose or stabilized modifications of those sugars), and one or more phosphate groups. Examples of modified phosphate groups are phosphorothioate or methylphosphonate. Each compound of the nucleotide is modifiable, and is naturally or non-naturally occurring. Examples of the latter are: locked nucleic acid (LNA), 2', 4' constrained ethyl nucleic acids (c-ET), 2'-0,4'-C-ethylene-bridged nucleic acid (ENA), polyalkylene oxide- (such as triethylene glycol (TEG)), 2'-fluoro-, 2'-deoxy-2'-fluoro-beta-D-arabinonucleic acid (FANA), 2'-0-methoxy- and 2'-O-methyl-modified nucleotides. Figure 3 shows examples of a number of different modified nucleotides that may be used in the context of the present invention.

An "LNA" is a modified RNA nucleotide, wherein the ribose moiety is modified with an extra bridge connecting the 2' oxygen and 4' carbon (2'- 4'ribonucleoside). The bridge locks the ribose in the 3'-endo (North) conformation, which is often found in the A-form duplexes. LNA nucleosides and nucleotides, respectively, comprise for example the forms of thio-LNA, oxy-LNA, or amino-LNA, in alpha-D- or beta-L-configuration, and can be mixed or combined, respectively, with DNA or RNA residues in the oligonucleotide.

A "bridged nucleic acid" is modified RNA nucleotide, sometimes also referred to as constrained or inaccessible RNA molecule, which may contain a five-membered, six-membered or even a seven-membered bridged structure with a "fixed" C3'-endo sugar puckering. The bridge is synthetically incorporated at the 2', 4'-position of the ribose to afford a 2', 4'-BNA monomer. Specific examples are "ENA" nucleotides, wherein the bridge is an ethylene bridge. Figure 3 shows a number of BNA nucleotides that may be used in the context of the present invention.

In a particular embodiment, one or more nucleotide(s) in said oligonucleotide are modified, wherein the modified nucleotide contains a modified phosphate groups, particularly selected from a phosphorothioate and a methylphosphonate, particularly a phosphorothioate. In particular embodiments, all phosphate groups of the oligonucleotide are modified phosphate groups, particularly independently selected from phosphorothioates and methylphosphonates, particularly wherein all phosphate groups are phosphorothioates.

In a particular embodiment, one or more nucleotide(s) in said oligonucleotide are modified, wherein the modified nucleotide is an LNA, a c-ET, an ENA, a polyalkylene oxide-, a 2'-fluoro-, a 2'-O-methoxy-, a FANA and/or a 2'-O-methyl-modified nucleotide.

In particular embodiments, the modified nucleotide(s) is/are located within the stretch of 5 nucleotides at the 5'- and/or 3'- end of the oligonucleotide, particularly at the 5'- and/or 3'-end of the oligonucleotide.

In particular embodiments, the oligonucleotides of the present invention comprise at least one modified nucleotide, particularly at least one LNA, c-ET and/or ENA, at the 5'- and/or 3'-end of the oligonucleotide. In a particular embodiment, the oligonucleotide comprises 1, 2, 3, or 4 LNAs or c-ETs or ENAs within the stretch of up to 5 nucleotides at the 5'-end, and 1, 2, 3, or 4 LNAs or c-ETs or ENAs within the stretch of up to 5 nucleotides at the 3 '-end. In another particular embodiment, the oligonucleotide comprises 1, 2, 3, or 4 LNAs, c-ETs, or ENAs at the within the stretch of 5 nucleotides 5'-end or 3'-end, and a polyalkylene oxide such as TEG within the stretch of 5 nucleotides at the 3'- or 5'-end.

In particular embodiments, said oligonucleotide is a Gapmer comprising at least one LNA nucleotide within the stretch of 5 nucleotides at the 5'-end of said oligonucleotide, and at least one LNA nucleotide within the stretch of 5 nucleotides at the 3'-end of said oligonucleotide. In particular embodiments, said Gapmer comprises 2 or 3 LNA nucleotides within the stretch of 5 nucleotides at the 5'-end of said oligonucleotide, and 2 or 3 LNA nucleotides within the stretch of 5 nucleotides at the 3'-end of said oligonucleotide.

In the context of the present invention, the term "Gapmer" refers to a chimeric antisense oligonucleotide that contains a central block of deoxynucleotide monomers sufficiently long to induce RNase H cleavage. The central block of a Gapmer is flanked by blocks of 2'-O modified ribonucleotides or other artificially modified ribonucleotide monomers such as bridged nucleic acids (BNAs) that protect the internal block from nuclease degradation. In many earlier studies modified DNA analogs were investigated for their stability in biological fluids. In the majority of these experiments phosphorothioate DNA analogs were used. More recently, several types of artificial nucleotide monomers including BNA monomers have been investigated for their usefulness in the design of Gapmers. Gapmers have been used to obtain RNase-H mediated cleavage of target RNAs, while reducing the number of phosphorothioate linkages. Phosphorothioates possess increased resistance to nucleases compared to unmodified DNA. However, they have several disadvantages. These include low binding capacity to complementary nucleic acids and non-specific binding to proteins that cause toxic side-effects limiting their applications. The occurrence of toxic side-effects together with non-specific binding causing off-target effects has stimulated the design of new artificial nucleic acids for the development of modified oligonucleotides that provide efficient and specific antisense activity in vivo without exhibiting toxic side-effects.

LNA Gapmers are powerful tools for loss of function studies of proteins, mRNA and IncRNAs. These single strand antisense oligonucleotides catalyze RNase H-dependent degradation of complementary RNA targets. LNA Gapmers are typically 12-20 nucleotides long enriched with LNA in the flanking regions and DNA in a LNA free central gap - hence the name Gapmer. The LNA-containing flanking regions confers nuclease resistance to the antisense oligo while at the same time increases target binding affinity regardless of the GC content. The central DNA "gap" activates RNase H cleavage of the target RNA upon binding.

In particular embodiments of the present in invention, the oligonucleotide comprises a sequence selected from the group consisting of SEQ ID NO. 2 to SEQ ID NO. 22, particularly from the group consisting of SEQ ID NO. 2 to SEQ ID NO. 13, more particularly from the group consisting of SEQ ID NO. 2 to SEQ ID NO. 10, more particularly from the group consisting of SEQ ID NO. 2 to SEQ ID NO. 9.

In a particular embodiment, the oligonucleotide is a variant of a sequence selected from the group consisting of SEQ ID NO. 2 to SEQ ID NO. 22, wherein in such variant one or more of the phosphorothioates are independently replaced by an unmodified phosphate of a modified phosphate other than a phosphorothioate, particularly a methylphosphonate

In a particular embodiment, the oligonucleotide is a variant of a sequence selected from the group consisting of SEQ ID NO. 2 to SEQ ID NO. 22, wherein such variant comprises one or more nucleotide mismatches particularly one or two mismatches, more particularly one mismatch, provided that any such variant including the mismatch(es), when analysed with the bioinformatic tools described in Example 1, contains at least one mismatch relative to human whole genome screening while maintaining homology to relevant species.

In a second aspect, the present invention relates to a pharmaceutical composition comprising an oligonucleotide according to the present invention.

In particular embodiments, the pharmaceutical composition further comprises a pharmaceutically acceptable carrier.

In particular embodiments, the pharmaceutical composition further comprises at least one additional component selected from: a further anti-sense compound, an antibody, a chemotherapeutic compound, an anti-inflammatory compound, an antiviral compound, an immuno-modulating compound, a pharmaceutically acceptable binding agents and an adjuvant.

In one embodiment, the oligonucleotide and the pharmaceutical composition, respectively, is formulated as dosage unit in form of capsules, tablets and pills etc., respectively, which contain for example the following compounds: microcrystalline cellulose, gum or gelatin as binders; starch or lactose as excipients; stearates as lubricants, various sweetening or flavouring agents. For capsules the dosage unit may contain a liquid carrier like fatty oils. Likewise coatings of sugar or enteric agents may be part of the dosage unit.

The oligonucleotide and/or the pharmaceutical composition is administrable via different routes. These routes of administration include, but are not limited to, electroporation, epidermal, impression into skin, intra-arterial, intra-articular, intracranial, intradermal, intralesional, intra-muscular, intranasal, intra-ocular, intrathecal, intracameral, intraperitoneal, intraprostatic, intrapulmonary, intraspinal, intratracheal, intratumoral, intravenous, intravesical, placement within cavities of the body, nasal inhalation, oral, pulmonary inhalation (e.g., by inhalation or insufflation of powders or aerosols, including by nebulizer), subcutaneous, subdermal, topical (including ophthalmic and to mucous membranes including vaginal and rectal delivery), or transdermal administration.

For parenteral, subcutaneous, intradermal or topical administration the oligonucleotide and/or the pharmaceutical composition include for example a sterile diluent, buffers, regulators of toxicity and antibacterials. In a preferred embodiment, the oligonucleotide or pharmaceutical composition is prepared with carriers that protect against degradation or immediate elimination from the body, including implants or microcapsules with controlled release properties. For intravenous administration the preferred carriers are for example physiological saline or phosphate buffered saline. An oligonucleotide and/or a pharmaceutical composition comprising such oligonucleotide for oral administration includes for example powder or granule, microparticulate, nanoparticulate, suspension or solution in water or non-aqueous media, capsule, gel capsule, sachet, tablet or minitablet. An oligonucleotide and/or a pharmaceutical composition comprising for parenteral, intrathecal, intracameral or intraventricular administration includes for example sterile aqueous solutions which optionally contain buffer, diluent and/or other suitable additive such as penetration enhancer, carrier compound and/or other pharmaceutically acceptable carrier or excipient.

A pharmaceutically acceptable carrier is for example liquid or solid, and is selected with the planned manner of administration in mind so as to provide for the desired bulk, consistency, etc., when combined with a nucleic acid and the other components of a given pharmaceutical composition. Typical pharmaceutically acceptable carriers include, but are not limited to, a binding agent (e.g. pregelatinized maize starch, polyvinylpyrrolidone or hydroxypropyl methylcellulose, etc.); filler (e.g. lactose and other sugars, microcrystalline cellulose, pectin, gelatin, calcium sulfate, ethyl cellulose, polyacrylates or calcium hydrogen phosphate, etc.); lubricant (e.g., magnesium stearate, talcum, silica, colloidal silicon dioxide, stearic acid, metallic stearates, hydrogenated vegetable oils, corn starch, polyethylene glycols, sodium benzoate, sodium acetate, etc.); disintegrate (e.g., starch, sodium starch glycolate, etc.); or wetting agent (e.g., sodium lauryl sulfate, etc.). Sustained release oral delivery systems and/or enteric coatings for orally administered dosage forms are described in U.S. Pat. Nos. 4,704,295; 4,556,552; 4,309,406; and 4,309,404. An adjuvant is included under these phrases.

Besides being used in a method of human disease prevention and/or treatment, the oligonucleotide and/or the pharmaceutical composition according to the present invention is also used in a method for prevention and/or treatment of other subjects including veterinary animals, reptiles, birds, exotic animals and farm animals, including mammals, rodents, and the like. Mammals include for example horses, dogs, pigs, cats, or primates (for example, a monkey, a chimpanzee, or a lemur). Rodents include for example rats, rabbits, mice, squirrels, or guinea pigs.

In a third aspect, the present invention relates to a pharmaceutical composition comprising the oligonucleotide according to the present invention for use in a method of preventing and/or treating a disease or disorder selected from the list of: an acute inflammatory disease, and a chronic inflammatory disease, a neurodegenerative disease, a malignant tumor, and a benign tumor.

In particular embodiments of the present invention, the inflammatory disease is selected from the group consisting of: psoriasis, rheumatoid arthritis (RA), Morbus Bechterew, multiple sclerosis (MS), systemic lupus erythematosus (SLE), Behcet's disease, uveitis, Sjögren syndrome, an inflammatory bowel disease (IBD), asthma, chronic obstructive pulmonary disease (COPD), neuropathic pain, atopic dermatitis, and allergy.

In particular other embodiments of the present invention, the tumor is selected from the group consisting of solid tumors, blood born tumors, leukemias, tumor metastasis, hemangiomas, acoustic neuromas, neurofibromas, trachomas, pyogenic granulomas, psoriasis, astrocytoma, acoustic neuroma, blastoma, Ewing's tumor, craniopharyngioma, ependymoma, medulloblastoma, glioma, hemangioblastoma, Hodgkin's lymphoma, medullablastoma, leukaemia, mesothelioma, neuroblastoma, neurofibroma, non-Hodgkin's lymphoma, pinealoma, retinoblastoma, sarcoma, seminoma, trachomas, and Wilms' tumor, or is selected from the group of bile duct carcinoma, bladder carcinoma, brain tumor, breast cancer, bronchogenic carcinoma, carcinoma of the kidney, cervical cancer, choriocarcinoma, choroid carcinoma, cystadenocarcinoma, embryonal carcinoma, epithelial carcinoma, esophageal cancer, cervical carcinoma, colon carcinoma, colorectal carcinoma, endometrial cancer, gallbladder cancer, gastric cancer, head cancer, liver carcinoma, lung carcinoma, medullary carcinoma, neck cancer, non-small-cell bronchogenic/lung carcinoma, ovarian cancer, pancreas carcinoma, papillary carcinoma, papillary adenocarcinoma, prostate cancer, small intestine carcinoma, prostate carcinoma, rectal cancer, renal cell carcinoma, retinoblastoma, skin cancer, small-cell bronchogenic/lung carcinoma, squamous cell carcinoma, sebaceous gland carcinoma, testicular carcinoma, and uterine cancer.

For the purpose of clarity and a concise description, features are described herein as part of the same or separate embodiments, however, it will be appreciated that the scope of the invention may include embodiments having combinations of all or some of the features described.

The following examples will serve to further illustrate the present invention without, at the same time, however, constituting any limitation thereof. On the contrary, it is to be clearly understood that the scope of the present invention refers to various other embodiments, modifications, and equivalents thereof which, after reading the description herein, may suggest themselves to those skilled in the art without departing from the spirit of the invention.

### Examples

### Example 1: Sequence selection and oligonucleotide modification process

At first suitable sequences representing possible target sites were identified using proprietary bioinformatics tools. In a second step the effects of chemical modifications on these sequences were predicted in silico to optimize modification patterns.

### 1. Stepwise Sequence Selection Process.

For RORC two isoforms that arise from differential promoter usage and thus differ in their first exon are known. These two transcript variants are annotated as: NM_005060 for ROR gamma (RORC1) and NM_001001523 for ROR gamma t (RORC2). The variant RORC2 differs in the 5' UTR and coding region compared to variant RORC1. Therefore isoform RORC2 is shorter and has a distinct N-terminus compared to isoform RORC1. The mRNAs of RORC2 (NM_001001523.1) and RORC1 (NM_005060) consist of 3054 nucleotides and 3084 nucleotides, respectively. Alignment of both sequences shows that only the first 148 nucleotides mostly located within the 5'untranslated region (UTR) are unique to RORC2 while the remaining nucleotides are shared.

Sequence lengths ranging from 13mer length to 17mer length in this region were analyzed (800 sequences in total) using proprietary bioinformatic tools.

Following factors were considered for selection of RORC2-specific antisense sequences:
- At least 3 mismatches to RORC1 mRNA
- No sequence should perfectly match (100%) any off-target mRNA in human
- Low number of partially homologous potential off-target mRNAs in human
- Cross-reactivity to mouse RORC2

### 2. Analysis of the effects of chemical modifications

The effects of chemical modifications on physicochemical properties such as melting temperature and tendency to form hairpins or dimers were evaluated using available prediction tools. Oligonucleotides with the most favorable predicted physicochemical properties were selected for synthesis and screen.

### Example2: Inhibition of RORC2 with antisense or DNAzyme constructs

### Material:

HDLM2 cell line - DSMZ (Deutsche Sammlung fur Mikroorganismen und Zelllinien, Braunschweig, Germany / ACC-17)

**Table 1. Machines used in the screening protocol**

| | Manufacturer | Serial Number |
|---|---|---|
| Centrifuge | Heraeus Megafuge 16R | 75004270 / 41826277 |
| Laminar Flow | ThermoFisher | S2020 1.2 / 41820790 |
| Heracell Vios 16oi Incubator | ThermoFisher | |
| Clariostar BMG | BMG | |

**Tab 2. Reagents used in the screening protocol**

| | Manufacturer | Serial Number | Lot Number |
|---|---|---|---|
| RPM11640 (fully supplemented) | 20150921 TE Labbook 1 | | |
| DEPC water | Ambion | AM9922 | 1506114 |
| 96wp flat bottom Nunclone Delta Surface | ThermoFisher | 167008 | 144112 |
| CellTiter Blue Reagent | Promega | G8088 | 152705 |
| Lysis Mixture | Affymetrix | 10093 | 2121507 |
| Proteinase K | Affymetrix | 12731 | 961504 |
| HPRT1 Probeset | Affymetrix | SA-10030 | 172411456 |
| RORC2 Probeset | Affymetrix | SA-50544 | 191082865 |

**Tab. 3: List of unmodified DNAzymes:**

| Name | Position on NM_001001523.1 | DNAzyme Sequence 5'-3' |
|---|---|---|
| D01001HM | 145-163 | TCACTTCAAGGCTAGCTACAACGATTGTGTTCT |
| D01002HM | 137-153 | TTGTGTTCTCAGGCTAGCTACAACGAGACTG |
| D01003H | 130-148 | TTCTCATGAGGCTAGCTACAACGATGAGCCTTG |
| D01004H | 2-20 | AGCTCTGCAGGCTAGCTACAACGACTAGCTCTC |

**Tab. 4. List of antisense oligonucleotides (+: LNA modification; *: PTO = phosphorothioate):**

| Name | Position on NM_001001 523.1 | mRNA Sequence 5'-3' | Antisense Sequence 5'-3' with PTO |
|---|---|---|---|
| A01001HM | 103-118 | AGGACAGGGAGCCAAG | +C*+T*T*G*G*C*T*C*C*C*T*G*T*+C*C*+T |
| A01002HM | 103-117 | AGGACAGGGAGCCAA | +T*+T*+G*G*C*T*C*C*C*T*G*T*+C*C*+T |
| A01003HM | 135-151 | CTCAGTCATGAGAACAC | +G*T*+G*+T*T*C*T*C* A*T*G*A*C*T*G*+A*+G |
| A01004HM | 136-151 | TCAGTCATGAGAACAC | +G*T*+G*T*T*C*T*C*A*T*G*A*C*+T*G*+A |
| A01005HM | 136-152 | TCAGTCATGAGAACACA | +T*G*T*+G*+T*T*C*T*C*A*T*G*A*C*+T*+G*+A |
| A01006HM | 136-151 | TCAGTCATGAGAACAC | +G*+T*+G*T*T*C*T*C*A*T*G*A*C+T*+G*+A |
| A01007HM | 137-151 | CAGTCATGAGAACAC | +G*+T*G*T*T*C*T*C*A*T*G*A*+C*T*+G |
| A01008HM | 138-152 | AGTCATGAGAACACA | +T*+G*+T*G*T*T*C*T*C*A*T*G*+A*+C*+T |
| A01009HM | 138-153 | AGTCATGAGAACACAA | +T*+T*G*+T*G*T*T*C*T*C*A*T*G*+A*+C*+T |
| A01010HM | 138-154 | AGTCATGAGAACACAAA | +T*+T*+T*G*T*G*T*T*C*T*C*A*T*G*+A*C*+T |
| A01011HM | 139-154 | GTCATGAGAACACAAA | +T*+T*+T*G*T*G*T*T*C*T*C*A*T*+G*+A*+C |
| A01012HM | 139-155 | GTCATGAGAACACAAAT | +A*+T*+T*T*G*T*G*T*T*C*T*C*A*T*+G*+A*+C |
| A01013HM | 140-155 | TCATGAGAACACAAAT | +A*+T*+T*T*G*T*G*T*T*C*T*C*A*T*+G*+A |
| A01014HM | 141-157 | CATGAGAACACAAATTG | +C*A*+A*+T*T*T*G*T*G*T*T*C+ T*C*+A*T*+G |
| A01015HM | 142-158 | ATGAGAACACAAATTGA | +T*+C*A*A*T*T*T*G*T*G*T*T*C*+T*+C*A*+T |
| A01016HM | 143-158 | TGAGAACACAAATTGA | +T*+C*A*+A*T*T*T*G*T*G*T*T*C*+T*+C*+A |
| A01017HM | 144-159 | GAGAACACAAATTGAA | +T*+T*+C*A*A*T*T*T*G*T*G*T*T*C*+T*+C |
| A01018HM | 145-159 | AGAACACAAATTGAA | +T*+T*+C*A*A*T*T*T*G*T*G*+T*+C*+T |
| A01019HM | 145-161 | AGAACACAAATTGAAGT | +A*+C*T*+T*C*A*A*T*T*T*G*T*G*T*+T*+C*+T |
| A01020HM | 146-161 | GAACACAAATTGAAGT | +A*+C*T*+T*C*A*A*T*T*T*G*T*G*+T*+T*+C |
| A01021HM | 146-162 | GAACACAAATTGAAGTG | +C*+A*+C*T*T*C*A*A*T*T*T*G*T*G*+T*+T*+C |
| A01022HM | 147-163 | AACACAAATTGAAGTGA | +T*+C*A*C*+T*T*C*A*A*T*T*T*G*T*+G*+ T*+T |
| A01023H | 4-19 | GAGCTAGGTGCAGAGC | +G*C*+T*C*T*G*C*A*C*C*T*A*G*C*+T*+C |
| A01024H | 6-22 | GCTAGGTGCAGAGCTTC | +G*+A*+A*G*C*T*C*T*G*C*A*C*C*T*+A*G*+C |
| A01025H | 7-22 | CTAGGTGCAGAGCTTC | +G*+A*+A*G*C*T*C*T*G*C*A*C*C*+T*+A*+G |
| A01026H | 7-21 | CTAGGTGCAGAGCTT | A*+A*+G*C*T*T*G*A*C*+C*T*+A |
| A01027H | 36-50 | GCTGAGAGGGCCTCG | +C*G*+A*G*G*C*C*C*T*C*T*C*+A*+G*+C |
| A01028H | 38-52 | TGAGAGGGCCTCGCC | +G*G*+C*G*A*G*G*C*C*C*T*C*+T*+C*+A |
| A01029H | 57-72 | CTCTGCCGCCAGCTGC | +G*C*+A*G*C*T*G*G*C*G*G*C*A*+G*+A*+G |

**Tab. 5: List of LNA - modified DNAzymes (+: LNA modification):**

| Name | Position on NM_001001523.1 | DNAzyme Sequence 5'-3' |
|---|---|---|
| D01005HM | 145-163 | +T+C+ACTTCAAGGCTAGCTACAACGATTGTGT+T+C+T |
| D01006HM | 145-163 | +TCACTT+CAAGGCTAGCTACAACGATT+GTGTTC+T |
| D01007HM | 140-160 | +CTT+CAAGGCTAGCTACAACGATT+G+TGTTCTCAT+G+A |
| D01008HM | 142-160 | +CTT+CAAGGCTAGCTACAACGATT+GTGTTCTCA+T |
| D01009HM | 146-161 | +ACTT+CAAGGCTAGCTACAACGATTGT+GTT+C |
| D01010HM | 137-153 | +TTGTGTTCTCAGGCTAGCTACAACGAG+ACT+G |
| D01011H | 130-148 | +T+TCTCATGAGGCTAGCTACAACGATGAG+CCTT+G |
| D01012H | 131-145 | +TC+ATGAGGCTAGCTACAACGATGAG+CCT+T |
| D01013H | 2-20 | +AGCT+CTGCAGGCTAGCTACAACGACTAGCTC+T+C |
| D01014H | 2-19 | +GCTCTGCAGGCTAGCTACAACGACTAGCTCT+C |
| D01015H | 3-19 | +GCTCTGCAGGCTAGCTACAACGACTAGCTC+T |
| D01016H | 4-19 | +GCTCTGCAGGCTAGCTACAACGACTAGCT+C |
| D01017H | 4-19 | +GCTC+TGCAGGCTAGCTACAACGACT+AGCT+C |

### Protocol:

HDLM2 cell line was purchased from DSMZ, expanded for master and working cell banks and cultured in supplemented RPMI 1640 medium (5% CO₂ and 37°C) for all further experiments. The cultivation periods of every thawed cell batch from the working cell bank were between two and three weeks.

All oligonucleotides were ordered from Exiqon (Vedbaek/Denmark) and Biospring (Frankfurt/ Germany). The lyophilized oligonucleotides were reconstituted with DEPC treated water up to concentration of 1 mM.

The initial screen was performed at single concentration of 10 µM for each nucleotide.

HDLM2 cells were plated out in supplemented RPM11640 at 15 000 cells / 50µl / well in 96 round bottom well plate. The oligonucleotides were diluted in supplemented RPM11640. For the starting concentration of 20 µM, 4 µl of oligonucleotide stock solution were diluted in 196 µl medium. Further, 50 µl of 20 µM (2x) oligonucleotide was added to each well of cells, leading to the final concentration of 10 µM. Each oligonucleotide was screened in triplicates (Figure 2A).

One scrambled (negative) control, Neg1 and cells without oligonucleotide treatment ("no oligo control") were used as controls. "No oligo control" triplicates were set up on each 96 well plate. In "no oligo controls" 50µl of supplemented medium were added. All remaining wells were filled with 150µl medium in order to prevent any evaporation effects.

Cells were incubated for 3 days at 37°C, 5% CO₂ without medium exchange. On day 3, a cell viability assay was performed in order to confirm the cell viability at the end of the screening experiment. For this 45 µl of each culture well were transferred into a new flat bottom 96 well plate and incubated with 9 µl Cell Titer Blue^{®} for 2 h at 37°C and 5% CO₂. The remaining 55 µl of each culture well were lysed for the mRNA quantification via bDNA assay. For the cell lysis each well was supplemented with 27µl working lysis mixture and incubated 30 min at 50-55°C.

The lysates were either used directly in the bDNA assay or frozen and stored away. The working lysis mixture is composed of lysis mixture and Proteinase K.

bDNA assay for HPRT1 and RORC2 was performed according to the manufacturer's protocol.

### Results:

The initial selection of antisense oligonucleotides in HDLM2 cells has resulted in several highly active molecules. The relative expressions on HPRT1 (housekeeping gene) and RORC2 have been calculated and plotted for all antisense oligonucleotides and untreated controls (Fig. 2A). Figure 2B shows the inhibition of expression of RORC2 relative to the expression of untreated control in HDLM2 cells.

**Tab. 6: Relative expression of HPRT1 and RORC2**

| | Relative expression (AU) | |
|---|---|---|
| | HPRT1 | RORC2 |
| A01018HM | | 0,01439834 |
| A01010HM | | 0,00909642 |
| A01011HM | 0,81641528 | 0,00743308 |
| A01017HM | 0,78598404 | 0,03196743 |
| A01019HM | 0,88045569 | 0,0327991 |
| A01021HM | 0,72101381 | 0,04454648 |
| A01016HM | 0,76828753 | 0,0750065 |
| A01007HM | 0,73860843 | 0,08242225 |
| A01012HM | 0,80949958 | 0,13682751 |
| A01015HM | 0,80366082 | 0,16354501 |
| A01013HM | 0,82306731 | 0,17248549 |
| A01004HM | 0,69563736 | 0,21500476 |
| A01006HM | 0,81490527 | 0,22827688 |
| A01009HM | 0,79101335 | 0,22841549 |
| A01003HM | 1,0249281 | 0,24969245 |
| A01005HM | 0,94096469 | 0,28510786 |
| A01001HM | 0,84362462 | 0,33386468 |
| A01020HM | 0,8135443 | 0,34640908 |
| A01014HM | 0,79889963 | 0,36966127 |
| A01022HM | 0,86216052 | 0,39436888 |
| A01024H | 0,80326214 | 0,40074504 |
| A01023H | 0,71017949 | 0,43425453 |
| A01029H | 0,89900051 | 0,44659101 |
| A01027H | 0,67353248 | 0,48775881 |
| A01028H | 0,70722954 | 0,59442086 |
| A01026H | 0,79550769 | 0,6371134 |
| A01008HM | 0,83706429 | 0,68417223 |
| A01025H | 0,95286894 | 0,7523694 |
| D01007HM | 0,76943643 | 0,7894828 |
| D01015H | 0,765991 | 0,83536342 |
| D01005HM | 0,95422673 | 0,83865546 |
| D01009HM | 0,85664274 | 0,88917959 |
| D01012H | 1,00375303 | 0,89642207 |
| D01013H | 0,9461252 | 0,91257039 |
| D01006HM | 0,83021164 | 0,91468422 |
| A01002HM | 0,95109464 | 0,92033267 |
| D01016H | 0,88074482 | 0,92220393 |
| D01011H | 0,96909112 | 1,04265789 |
| D01014H | 0,93413561 | 1,12582518 |
| D01010HM | 0,94777149 | 1,17163649 |
| D01017H | 1,02497077 | 1,41101967 |
| D01008HM | 0,91486541 | 1,43104912 |
| no oligo 1 | 0,96957896 | 1,21730919 |
| no oligo 2 | 1,10286104 | 1,15448324 |
| no oligo 3 | 1,00015794 | 1,1734731 |
| neg1 | 1 | 1 |

**Tab. 7: Sequence ID Nos:**

| **SEQ ID No:** | **Sequence identifier** |
|---|---|
| 1 | RORC2 sequence (see Fig. 1) |
| 2 | A01018HM |
| 3 | A01010HM |
| 4 | A01011HM |
| 5 | A01017HM |
| 6 | A01019HM |
| 7 | A01021HM |
| 8 | A01016HM |
| 9 | A01007HM |
| 10 | A01012HM |
| 11 | A01015HM |
| 12 | A01013HM |
| 13 | A01003HM |
| 14 | A01006HM |
| 15 | A01009HM |
| 16 | A01005HM |
| 17 | A01004HM |
| 18 | A01001HM |
| 19 | A01020HM |
| 20 | A01022HM |
| 21 | A01014HM |
| 22 | A01029HM |

## Claims

1. An oligonucleotide consisting of from 10 to 20 nucleotides, particularly from 15 to 18 nucleotides, wherein the sequence of said oligonucleotide corresponds to the antisense strand of the RORC2 nucleic acid coding sequence of SEQ ID NO. 1, wherein one or more nucleotide(s) of the oligonucleotide is/are optionally modified, and wherein said oligonucleotide inhibits the expression of RORC2 relative to the expression of untreated control in HDLM2 cells by at least 50%.

2. The oligonucleotide of claim 1, wherein said oligonucleotide inhibits the expression of RORC2 relative to the expression HPRT1 in HDLM2 cells by at least 75%, particularly by at least 80%, more particularly by at least 85%.

3. The oligonucleotide of claim 1 or 2, wherein one or more nucleotide(s) in said oligonucleotide are modified, wherein the modified nucleotide is an LNA, a c-ET, an ENA, a polyalkylene oxide-, a 2'-fluoro-, a 2'-O-methoxy-, a FANA and/or a 2'-O-methyl-modified nucleotide.

4. The oligonucleotide of claim 3, wherein the modified nucleotide(s) is/are located within the stretch of 5 nucleotides at the 5'- and/or 3'- end of the oligonucleotide, particularly at the 5'- and/or 3'- end of the oligonucleotide

5. The oligonucleotide according to claim 4, wherein said oligonucleotide is a Gapmer comprising at least one LNA nucleotide within the stretch of 5 nucleotides at the 5'-end of said oligonucleotide, and at least one LNA nucleotide within the stretch of 5 nucleotides at the 3'-end of said oligonucleotide.

6. The oligonucleotide according to any one of claims 1 to 5, wherein the oligonucleotide comprises a sequence selected from the group consisting of SEQ ID NO. 2 to SEQ ID NO. 22, particularly from the group consisting of SEQ ID NO. 2 to SEQ ID NO. 13, more particularly from the group consisting of SEQ ID NO. 2 to SEQ ID NO. 10, more particularly from the group consisting of SEQ ID NO. 2 to SEQ ID NO. 9.

7. Pharmaceutical composition comprising the oligonucleotide according to any one of claims 1 to 6.

8. The oligonucleotide according to any one of claims 1 to 6 or the pharmaceutical composition according to claim 7 for use in a method of preventing and/or treating a disease or disorder selected from the list of: an acute inflammatory disease, a chronic inflammatory disease, a neurodegenerative disease, a malignant tumor, and a benign tumor.

9. The oligonucleotide or pharmaceutical composition for use according to claim 8, wherein the inflammatory disease is selected from the group consisting of: psoriasis, rheumatoid arthritis (RA), Morbus Bechterew , multiple sclerosis (MS), systemic lupus erythematosus (SLE), Behcet's disease, uveitis, Sjögren syndrome, an inflammatory bowel disease (IBD), asthma, chronic obstructive pulmonary disease (COPD), neuropathic pain, atopic dermatitis, and allergy.

10. The oligonucleotide or pharmaceutical composition for use according to claim 8, wherein the tumor is selected from the group consisting of solid tumors, blood born tumors, leukemias, tumor metastasis, hemangiomas, acoustic neuromas, neurofibromas, trachomas, pyogenic granulomas, psoriasis, astrocytoma, acoustic neuroma, blastoma, Ewing's tumor, craniopharyngioma, ependymoma, medulloblastoma, glioma, hemangioblastoma, Hodgkin's lymphoma, medullablastoma, leukaemia, mesothelioma, neuroblastoma, neurofibroma, non-Hodgkin's lymphoma, pinealoma, retinoblastoma, sarcoma, seminoma, trachomas, and Wilms' tumor, or is selected from the group consisting of bile duct carcinoma, bladder carcinoma, brain tumor, breast cancer, bronchogenic carcinoma, carcinoma of the kidney, cervical cancer, choriocarcinoma, choroid carcinoma, cystadenocarcinoma, embryonal carcinoma, epithelial carcinoma, esophageal cancer, cervical carcinoma, colon carcinoma, colorectal carcinoma, endometrial cancer, gallbladder cancer, gastric cancer, head cancer, liver carcinoma, lung carcinoma, medullary carcinoma, neck cancer, non-small-cell bronchogenic/lung carcinoma, ovarian cancer, pancreas carcinoma, papillary carcinoma, papillary adenocarcinoma, prostate cancer, small intestine carcinoma, prostate carcinoma, rectal cancer, renal cell carcinoma, retinoblastoma, skin cancer, small-cell bronchogenic/lung carcinoma, squamous cell carcinoma, sebaceous gland carcinoma, testicular carcinoma, and uterine cancer.
